# FASCICULE DE BREVET EUROPEEN

(11) **EP 0 801 639 B1**
(45) Date de publication et mention de la délivrance du brevet: **02.01.2002**
(21) Numéro de dépôt: 96937374.5
(22) Date de dépôt: 31.10.1996
(51) Int. Cl.: C07C 46/00, C07C 50/34, C07C 69/013

(54) **PROCEDE DE PREPARATION D'ANTHRAQUINONES SUBSTITUEES ET APPLICATION A LA PREPARATION DE RHEINES**
VERFAHREN ZUR HERSTELLUNG VON SUBSTITUIERTEN ANTHRACHINONEN UND DEREN VERWENDUNG ZUR HERSTELLUNG VON RHEINEN
METHOD FOR PREPARING SUBSTITUTED ANTHRAQUINONES AND USE THEREOF FOR PREPARING RHEINS

(30) Priorité: 02.11.1995 FR 9512950
(43) Date de publication de la demande: 22.10.1997
(73) Titulaire: Girex, 29000 Quimper (FR)
(72) Inventeur: ESTANOVE, Cyril, F-92100 Boulogne (FR); PRUVOST, François, F-29000 Quimper (FR)
(74) Mandataire: L'Helgoualch, Jean
(86) Numéro de dépôt international: FR9601714
(87) Numéro de publication internationale: WO9716404

(56) Documents cités:
- DE-A- 2 144 772
- JOURNAL OF THE AMERICAN CHEMICAL SOCIETY, vol. 99, no. 24, 23 Novembre 1977, DC US, pages 8116-8118, XP002006815 BARRY M. TROST ET AL.: "On the Regioselectivity of the Catalyzed and Uncatalyzed Diels-Alder Reaction"
- JOURNAL OF ORGANIC CHEMISTRY, vol. 53, no. 17, 19 Août 1988, EASTON US, pages 4052-4059, XP002006816 LOUISE BOISVERT ET AL.: "Regiospecific Addition of Monooxygenated Dienes to Halo Quinones"

## Description

La présente invention concerne un nouveau procédé de préparation d'anthraquinones substituées à partir de 1,4-naphtoquinones, ainsi que l'application des produits obtenus comme intermédiaires de synthèse de rhéines présentant des propriétés utiles en thérapeutique.

La préparation d'anthraquinones telles que le chrysophanol par addition de 6-méthoxy-4-méthyl-pyrone sur une naphtoquinone telle que la juglone, suivant la réaction de Diels-Alder, a été décrite par M. E. Jung et al., J.C.S. Chem. Comm. 95 (1978). Cependant, ce procédé nécessite plusieurs étapes, c'est-à-dire une addition suivie d'une oxydation par l'oxyde d'argent pour provoquer l'aromatisation des cycles, et une déméthylation. De plus, la réaction implique l'utilisation de diazométhane qui présente des inconvénients bien connus.

Des voies de synthèses d'anthracyclinones par réaction de cyclo-addition de Diels-Alder ont aussi été décrites par M. Petrzilka et J.I. Grayson [Synthesis, 753 (1981)]. Suivant ces auteurs, la réaction d'addition régio-spécifique d'un diène sur une quinone peut être obtenue en utilisant comme catalyseur un acide de Lewis constitué par le composé BF₃-O(C₂H₅)₂.

Le brevet DE-A-2144772 décrit un procédé de préparation de 1-hydroxy-3-méthyl-anthraquinones par réaction d'un dialcoxy-méthyl-butadiène sur une naphtoquinone halogénée en position 2. La réaction est effectuée en deux étapes, par condensation puis désalkylation.

J. of Org. Chem. 53, (1988) p. 4052-4059 (L. Boisvert et al.) décrit l'addition d'acétoxy-butadiènes sur des quinones halogénées par réaction de Diels-Alder. Les naphtoquinones obtenues peuvent ensuite être transformées en anthraquinones.

Le procédé suivant la présente invention permet de préparer des anthraquinones substituées à partir de 1,4-naphtoquinones, en seulement deux étapes et avec un rendement satisfaisant.

Les anthraquinones que l'on peut préparer par le procédé suivant la présente invention peuvent être représentées par la formule générale (I) ci-après : 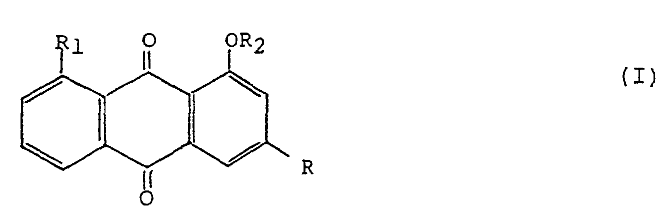 dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est atome d'hydrogène ou un groupe alkyle linéaire ou ramifié de 1 à 5 atomes de carbone, R₁ représente un atome d'hydrogène, un groupe hydroxy, alcoxy linéaire ou ramifié de 1 à 5 atomes de carbone, ou acyloxy de 1 à 5 atomes de carbone, et R₂ représente un atome d'hydrogène.

Conformément au procédé de l'invention, on effectue une réaction de Diels-Alder entre une 1,4-naphtoquinone de formule générale (II) : 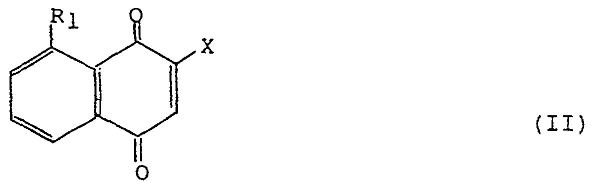
dans laquelle R₁ représente un atome d'hydrogène, un groupe hydroxy, alcoxy linéaire ou ramifié de 1 à 5 atomes de carbone, ou acyloxy de 1 à 5 atomes de carbone, et X représente un atome d'hydrogène ou un atome d'halogène,
   et un diène acyclique de formule (III) :

      CH₂ = CR - CH = CH - OR₃ (III)
dans laquelle R représente un atome d'hydrogène ou un groupe alkyle linéaire ou ramifié de 1 à 5 atomes de carbone, un un groupe chlorométhyle, un groupe -COCl, un groupe -COOR' ou un groupe -CH2OR' où R' est un atome d'hydrogène ou un groupe alkyle linéaire ou ramifié de 1 à 5 atomes de carbone, et R3 représente un groupe acétyle, en présence d'hydroquinone en quantité catalytique,
   et on effectue ensuite une réaction de saponification, d'aromatisation et d'oxydation.

Dans la formule (II) ci-dessus représentant la naphtoquinone de départ, de préférence, R1 représente un groupe hydroxy ou acétoxy, et X est un atome d'hydrogène ou un atome de chlore. Dans la formule générale (III) représentant le diène acyclique, il est préférable que R représente un atome d'hydrogène ou un groupe méthyle.

Le diène acyclique de formule (III) utilisé dans la réaction décrite ci-dessus peut être par exemple le 1-acétoxy-1,3-butadiène ou le 1-acétoxy-3-méthyl-1,3-butadiène.

Parmi les naphtoquinones de formule générale (II), on utilise de préférence la juglone, représentée par la formule (II) où R1 représente un groupe hydroxyle, ou la 3-chlorojuglone, représentée par la même formule où X est un atome de chlore. La juglone peut être préparée par exemple par oxydation du 1,5-dioxynaphtalène en présence d'un catalyseur approprié comme décrit dans le brevet SU-1.817.767, ou par l'oxyde de chrome par la méthode de G. Jesaitis et al., J. Chem. Ed., 49, 436 (1972), ou encore par oxydation au moyen de l'oxygène de l'air en présence d'un catalyseur à base de cobalt tel que la salcomine, suivant la méthode de T. Wakamatsu et al., Synthetic Communications, 14, 1167 (1984).

La réaction de cyclo-addition de Diels-Alder entre la 1,4-naphtoquinone de formule générale (II) et le diène acyclique de formule générale (III) s'effectue de préférence dans un solvant qui peut être choisi parmi les solvants hydrocarbonés et les alcools, tel que le toluène, le xylène, le benzène ou le méthanol, en présence d'anhydride acétique. L'anhydride acétique est de préférence utilisé en excès. La réaction est effectuée en présence d'hydroquinone en quantité catalytique.

Suivant une variante de mise en oeuvre de l'invention, la réaction est effectuée en présence d'un acide de Lewis, qui peut être choisi de préférence parmi le chlorure de zinc et le chlorure ferrique.

La réaction d'addition peut s'effectuer à température ambiante ou en chauffant légèrement à une température comprise entre 20 et 60°C. On peut également opérer en chauffant à la température de reflux du solvant, par exemple, à une température comprise entre 60 et 130°C environ, suivant le solvant utilisé.

Le procédé conforme à la présente invention est particulièrement avantageux en ce qu'il permet d'obtenir l'anthraquinone, c'est-à-dire un composé à cycle aromatique, de manière simple en deux étapes sans qu'il soit nécessaire d'isoler des composés intermédiaires et sans utilisation de composé tel que l'oxyde d'argent pour provoquer l'aromatisation, contrairement aux schémas réactionnels classiques.

La réaction de cyclo-addition fournit les tétrahydroanthraquinones correspondant aux anthraquinones de formule (I) sous forme de mélange de deux isomères où le groupe R est en position 2 ou 3 et le groupe OR₃ en position 4 ou 1, respectivement, dans des proportions variables suivant les conditions opératoires. Ces tétrahydroanthraquinones peuvent être aisément transformées en anthraquinones de formule (I) par saponification, oxydation et aromatisation, par exemple par traitement par un oxydant tel que le N-bromosuccinimide ou le dioxyde de manganèse. Suivant une forme avantageuse de réalisation, on effectue la réaction de saponification-oxydation - aromatisation directement sur le mélange des tétrahydroanthraquinones isomères, ce qui permet de faciliter le traitement et d'améliorer le rendement global.

Les anthraquinones substituées obtenues par le procédé suivant la présente invention peuvent être utilisées dans la préparation de rhéines de formule générale (IV) 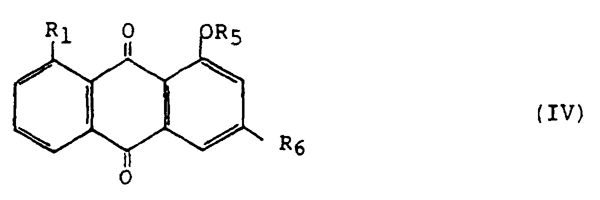 dans laquelle R₅ représente un groupe acétyle et R₆ représente un groupe -CO₂R' où R' est un atome d'hydrogène ou un groupe alkyle linéaire ou ramifié de 1 à 5 atomes de carbone, que l'on obtient en effectuant une acétylation des anthraquinones substituées de formule générale (I), suivie si nécessaire d'une oxydation et d'une purification.

Ces rhéines sont utiles en thérapeutique humaine et vétérinaire comme principes actifs de médicaments, notamment comme anti-inflammatoires non stéroïdiens pour le traitement de l'arthrite et de l'arthrose.

Les exemples suivants illustrent l'invention plus en détail sans en limiter la portée.

### Exemple 1

Dans un ballon de 100 ml, on porte à reflux (environ 110°C) un mélange de 0,5 g de juglone, 0,93 g de 1-acétoxy-3-méthyl-1,3-butadiène et 0,9 ml d'anhydride acétique dans 10 ml de toluène, et on maintient à reflux pendant 48 heures, en présence d'hydroquinone en quantité catalytique.

L'évolution de la réaction est suivie par chromatographie sur couche mince. La réaction est terminée lorsque la juglone n'est plus détectée.

Le toluène est éliminé par distillation sous pression réduite, et le produit brut obtenu est chromatographié sur colonne de gel de silice en éluant par un mélange cyclohexane / éther éthylique (80 / 20).

On obtient ainsi 180 mg de mélange constitué de 65% de 1-acétoxy-8-hydroxy-3-méthyl-1,1a,4,4a-tétrahydroanthraquinone et 35% de 4-acétoxy-8-hydroxy-2-méthyl-1,1a,4,4a-tétrahydroanthraquinone.

Les deux isomères sont rapidement saponifiés en milieu basique (carbonate de sodium dilué), oxydés et aromatisés à l'aide de 150 mg de dioxyde de manganèse(IV). Les deux isomères sont séparés par chromatographie sur colonne de gel de silice. Le chrysophanol ainsi obtenu avec un rendement de 9% est identifié par son spectre de RMN.

### Exemple 2

On procède comme dans l'Exemple 1, mais en utilisant 5,4 ml d'anhydride acétique dans le toluène, et en chauffant à reflux pendant 24 heures.

La réaction est complète au bout d'environ 24 heures. Le toluène est éliminé par distillation sous pression réduite.

On obtient ainsi 200 mg de mélange constitué de 65% de 1-acétoxy-8-hydroxy-3-méthyl-1,1a,4,4a-tétrahydroanthraquinone et 35% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 10%.

### Exemple 3

On procède comme dans l'Exemple 2, mais en remplaçant le toluène par du xylène.

La réaction se déroule de la même manière et on obtient 200 mg de mélange constitué de 65% de 1-acétoxy-8-hydroxy-3-méthyl-1,1a,4,4a-tétrahydroanthraquinone et 35% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 10%.

### Exemple 4

Dans un ballon de 100 ml, on porte à reflux un mélange de 0,5 g de juglone, 0,93 g de 1-acétoxy-3-méthyl-1,3-butadiène et 195 mg de chlorure de zinc dans 10 ml de toluène. Le mélange est maintenu à reflux pendant 90 minutes environ, en présence d'hydroquinone en quantité catalytique.

La juglone n'est plus détectée par chromatographie sur couche mince au bout de 90 minutes.

Le toluène est éliminé par distillation sous pression réduite, et le produit brut obtenu est chromatographié sur colonne de gel de silice en éluant par un mélange cyclohexane / éther éthylique (80 / 20).

On obtient ainsi 210 mg de mélange constitué de 70% de 1-acétoxy-8-hydroxy-3-méthyl-1,1a,4,4a-tétrahydroanthraquinone et 30% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 11%.

### Exemple 5

Dans un ballon de 100 ml, on mélange 0,5 g de juglone et 0,93 g de 1-acétoxy-3-méthyl-1,3-butadiène dans 10 ml de toluène. Le mélange est maintenu à température ambiante sous agitation pendant 14 heures environ, en présence d'hydroquinone en quantité catalytique.

La juglone n'est plus détectée par chromatographie sur couche mince au bout de 14 heures.

Le toluène est éliminé par distillation sous pression réduite, et le produit brut obtenu est chromatographié sur colonne de gel de silice en éluant par un mélange cyclohexane / éther éthylique (80 / 20).

On obtient ainsi 670 mg de mélange constitué de 80% de 1-acétoxy-8-hydroxy-3-méthyl-1,1a,4,4a-tétrahydroanthraquinone et 20% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 42%.

### Exemple 6

On procède comme dans l'Exemple 4, en utilisant 78 mg de chlorure de zinc, mais en laissant la réaction s'effectuer à température ambiante, sous agitation, en présence d'hydroquinone en quantité catalytique.

Le contrôle par chromatographie sur couche mince montre que la juglone a réagi au bout de 14 heures.

Le produit recherché est recueilli après distillation du toluène sous pression réduite et chromatographie sur colonne de gel de silice en éluant par un mélange cyclohexane / éther éthylique (80 / 20).

On obtient ainsi 700 mg de mélange constitué de 70% de 1-acétoxy-8-hydroxy-3-méthyl-1,1a,4,4a-tétrahydroanthraquinone et 30% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 38%.

### Exemple 7

On procède comme dans l'Exemple 6, mais en remplaçant le chlorure de zinc par 93 mg de chlorure ferrique dans 10 ml de toluène, à température ambiante sous agitation.

La juglone de départ n'est plus détectée par chromatographie sur couche mince au bout de 14 heures.

Après distillation du toluène sous pression réduite, le produit brut est chromatographié sur colonne de gel de silice en éluant avec un mélange cyclohexane / éther éthylique (80 / 20).

On obtient ainsi 710 mg de mélange constitué de 55% de 1-acétoxy-8-hydroxy-3-méthyl-1,1a,4,4a-tétrahydroanthraquinone et 45% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 30%.

### Exemple 8

On procède comme dans l'Exemple 5 en faisant réagir un mélange de 0,6 g de 3-chlorojuglone et 0,93 g de 1-acétoxy-3-méthyl-1,3-butadiène dans 10 ml de toluène.

La juglone n'est plus détectée par chromatographie sur couche mince au bout de 14 heures, ce qui montre que la réaction est terminée.

Le toluène est éliminé par distillation sous pression réduite, et le produit brut obtenu est chromatographié sur colonne de gel de silice en éluant par un mélange cyclohexane / éther éthylique (80 / 20).

On obtient ainsi 660 mg de mélange constitué de 90% de 1-acétoxy-8-hydroxy-3-méthyl-1,1a,4,4a-tétrahydroanthraquinone et 10% de 4-acétoxy-8-hydroxy-2-méthyl-1,1a,4,4a-tétrahydroanthraquinone.

Après saponification, oxydation et aromatisation suivant la technique décrite dans l'Exemple 1, on obtient le chrysophanol avec un rendement de 46%.

### Exemple 9

On procède comme dans l'Exemple 5 en faisant réagir un mélange de 0,5 g de juglone et 0,83 g de 1-acétoxy-1,3-butadiène dans 10 ml de toluène.

La juglone n'est plus détectée par chromatographie sur couche mince au bout de 12 heures.

Le toluène est éliminé par distillation sous pression réduite, et le produit brut obtenu est chromatographié sur colonne de gel de silice en éluant par un mélange cyclohexane / éther éthylique (80 / 20).

On obtient ainsi 660 mg de mélange constitué de 80% de 1-acétoxy-8-hydroxy-1,1a,4,4a-tétrahydroanthraquinone et 20% de 4-acétoxy-8-hydroxy-1,1a,4,4a-tétrahydroanthraquinone.

Après saponification, oxydation et aromatisation suivant la technique décrite dans l'Exemple 1, on obtient la 1,8-dihydroxyanthraquinone avec un rendement de 41%.

## Revendications

1. Procédé de préparation d'anthraquinones substituées représentées par la formule générale (I) ci-dessous 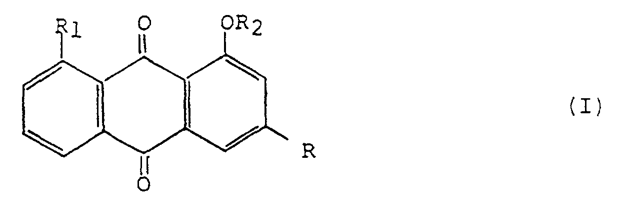
dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est atome d'hydrogène ou un groupe alkyle linéaire ou ramifié de 1 à 5 atomes de carbone, R₁ représente un atome d'hydrogène, un groupe hydroxyle, alcoxy linéaire ou ramifié de 1 à 5 atomes de carbone, ou acyloxy de 1 à 5 atomes de carbone, et R₂ représente un atome d'hydrogène,
**caractérisé en ce que** l'on effectue une réaction de Diels-Alder entre une 1,4-naphtoquinone de formule générale (II) : 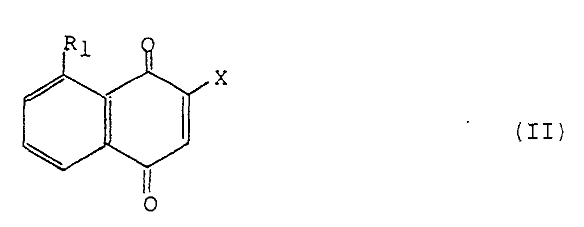
dans laquelle R₁ représente un atome d'hydrogène, un groupe hydroxy, alcoxy linéaire ou ramifié de 1 à 5 atomes de carbone, ou acyloxy de 1 à 5 atomes de carbone, et X représente un atome d'hydrogène ou un atome d'halogène,
et un diène acyclique de formule (III) :
CH₂ = CR - CH = CH - OR₃ (III)
dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est un atome d'hydrogène ou un groupe alkyle linéaire ou ramifié de 1 à 5 atomes de carbone, et R₃ représente un groupe acétyle, en présence d'hydroquinone en quantité catalytique,
suivie d'une réaction de saponification, d'aromatisation et d'oxydation,
pour obtenir. l'anthraquinone substituée de formule générale (I).

2. Procédé selon la revendication 1, **caractérisé en ce que** la réaction est effectuée en présence d'anhydride acétique.

3. Procédé selon l'une quelconque des revendications 1 et 2, **caractérisé en ce que** la réaction est effectuée en présence d'un acide de Lewis.

4. Procédé selon la revendication 3, **caractérisé en ce que** l'acide de Lewis est choisi parmi le chlorure de zinc et le chlorure ferrique.

5. Procédé selon l'une quelconque des revendications précédentes, **caractérisé en ce que** R₁ représente un groupe hydroxyle ou acétoxy, et X est un atome d'hydrogène ou un atome de chlore.

6. Procédé selon l'une quelconque des revendications précédentes, **caractérisé en ce que** R représente un atome d'hydrogène ou un groupe méthyle.

7. Procédé selon la revendication 6, **caractérisé en ce que** le diène de formule (III) est choisi parmi le 1-acétoxy-1,3-butadiène ou le 1-acétoxy-3-méthyl-1,3-butadiène.

8. Procédé selon la revendication 1, **caractérisé en ce que** l'on effectue une acétylation des anthraquinones substituées obtenues, suivie si nécessaire d'une oxydation, pour obtenir les rhéines de formule générale (IV) 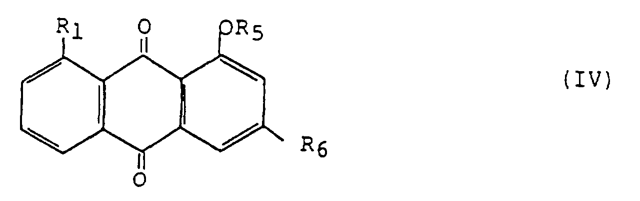 dans laquelle R₅ représente un groupe acétyle et R₆ représente un groupe -CO₂R' où R' est un atome d'hydrogène ou un groupe alkyle linéaire ou ramifié de 1 à 5 atomes de carbone.

## Patentansprüche

1. Verfahren zur Herstellung von substituierten Anthrachinonen der folgenden allgemeinen Formel (I): 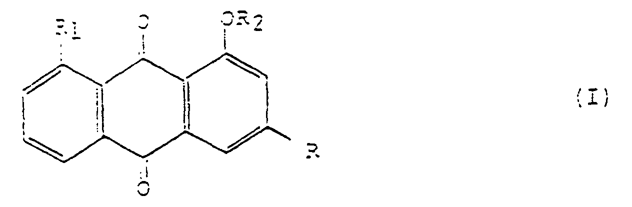 worin R für ein Wasserstoffatom oder eine unverzweigte oder verzweigte Alkylgruppe mit 1 bis 5 Kohlenstoffatomen, eine Chlormethylgruppe, eine Gruppe -COCl, eine Gruppe -COOR' oder eine Gruppe -CH₂OR' steht, worin R' ein Wasserstoffatom oder eine unverzweigte oder verzweigte Alkylgruppe mit 1 bis 5 Kohlenstoffatomen ist, R₁ für ein Wasserstoffatom, eine Hydroxylgruppe, unverzweigtes oder verzweigtes Alkoxy mit 1 bis 5 Kohlenstoffatomen oder Acyloxy mit 1 bis 5 Kohlenstoffatomen steht, und R₂ für ein Wasserstoffatom steht,
**dadurch gekennzeichnet, dass** eine Diels-Alder-Reaktion zwischen einem 1,4-Naphthochinon der allgemeinen Formel (II): 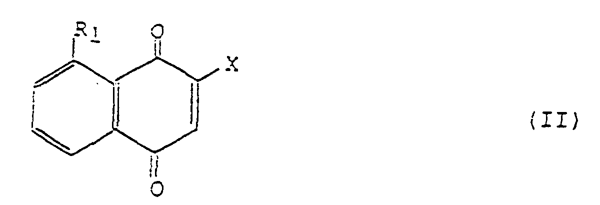 worin R₁ für ein Wasserstoffatom, eine Hydroxygruppe, unverzweigtes oder verzweigtes Alkoxy mit 1 bis 5 Kohlenstoffatomen oder Acyloxy mit 1 bis 5 Kohlenstoffatomen steht, und X für ein Wasserstoffatom oder ein Halogenatom steht.
und einem azyklischen Dien der Formel (III):
CH₂ = CR - CH = CH - OR₃ (III)
worin R für ein Wasserstoffatom oder eine unverzweigte oder verzweigte Alkylgruppe mit 1 bis 5 Kohlenstoffatomen, eine Chlormethylgruppe, eine Gruppe -COCl, eine Gruppe -COOR' oder eine Gruppe -CH₂OR' steht, worin R' ein Wasserstoffatom oder eine unverzweigte oder verzweigte Alkylgruppe mit 1 bis 5 Kohlenstoffatomen ist, und R₁ eine Acetylgruppe darstellt, in Gegenwart von Hydrochinon in katalytischen Mengen durchgeführt wird,
gefolgt von einer Verseifungs-, Aromatisierungs- und Oxidationsreaktion,
um das substituierte Anthrachinon der allgemeinen Formel (I) zu erhalten.

2. Verfahren nach Anspruch 1, **dadurch gekennzeichnet, dass** die Reaktion in Gegenwart von Essigsäureanhydrid durchgeführt wird.

3. Verfahren nach einem der Ansprüche 1 und 2, **dadurch gekennzeichnet, dass** die Reaktion in Gegenwart einer Lewis-Säure durchgeführt wird.

4. Verfahren nach Anspruch 3, **dadurch gekennzeichnet. dass** die Lewis-Säure aus Zinkchlorid und Eisen(III)-Chlorid ausgewählt ist.

5. Verfahren nach einem der vorangegangenen Ansprüche, **dadurch gekennzeichnet, dass** R₁ für eine Hydroxyl- oder Acetoxygruppe steht und X ein Wasserstoffatom oder ein Chloratom ist.

6. Verfahren nach einem der vorangegangenen Ansprüche, **dadurch gekennzeichnet, dass** R ein Wasserstoffatom oder eine Methylgruppe darstellt.

7. Verfahren nach Anspruch 6, **dadurch gekennzeichnet, dass** das Dien der Formel (III) aus 1-Acetoxy-1,3-butadien oder 1-Acetoxy-3-methyl-1,3-butadien ausgewählt ist.

8. Verfahren nach Anspruch 1, **dadurch gekennzeichnet, dass** eine Acetylierung der erhaltenen substituierten Anthrachinone, falls notwendig gefolgt von einer Oxidation, durchgeführt wird, um die Rheine der allgemeinen Formel (IV) 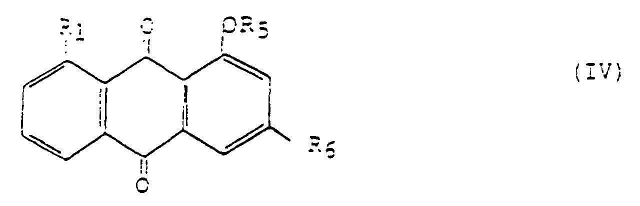 zu erhalten, worin R₅ für eine Acetylgruppe steht und R₆ für eine Gruppe -CO₂R' steht, worin R' ein Wasserstoffatom oder eine unverzweigte oder verzweigte Alkylgruppe mit 1 bis 5 Kohlenstoffatomen ist.

## Claims

1. Process for the preparation of substituted anthraquinones represented by the general formula (I) below 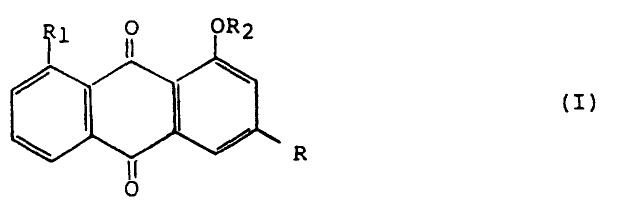 in which R represents a hydrogen atom or a linear or branched alkyl group containing 1 to 5 carbon atoms, a chloromethyl group, a -COCl group, a -COOR' group or a -CH₂OR' group where R' is a hydrogen atom or a linear or branched alkyl group containing 1 to 5 carbon atoms, R₁ represents a hydrogen atom, a hydroxyl group, a linear or branched alkoxy group containing 1 to 5 carbon atoms or an acyloxy group containing 1 to 5 carbon atoms, and R₂ represents a hydrogen atom,
**characterized in that** a Diels-Alder reaction is carried out between a 1,4-naphthoquinone of general formula (II): 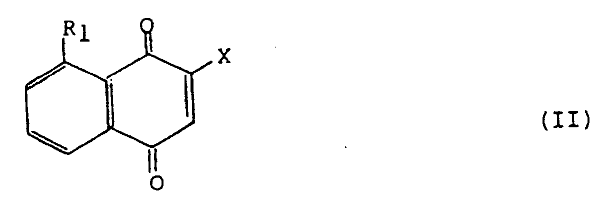 in which R₁ represents a hydrogen atom, a hydroxyl group, a linear or branched alkoxy group containing 1 to 5 carbon atoms or an acyloxy group containing 1 to 5 carbon atoms, and X represents a hydrogen atom or a halogen atom,
and an acyclic diene of formula (III):
CH₂ = CR - CH = CH - OR₃ (III)
in which R represents a hydrogen atom or a linear or branched alkyl group containing 1 to 5 carbon atoms, a chloromethyl group, a -COCl group, a -COOR' group or a -CH₂OR' group where R' is a hydrogen atom or a linear or branched alkyl group containing 1 to 5 carbon atoms and R₃ represents a acetyl group, in the presence of a catalytic amount of hydroquinone,
followed by a saponification, aromatization and oxidation reaction,
in order to obtain the substituted anthraquinone of general formula (I).

2. Process according to Claim 1, **characterized in that** the reaction is carried out in the presence of acetic anhydride.

3. Process according to either one of Claims 1 and 2, **characterized in that** the reaction is carried out in the presence of a Lewis acid.

4. Process according to Claim 3, **characterized in that** the Lewis acid is chosen from zinc chloride and ferric chloride.

5. Process according to any one of the preceding claims, **characterized in that** R₁ represents a hydroxyl or acetoxy group and X is a hydrogen atom or a chlorine atom.

6. Process according to any one of the preceding claims, **characterized in that** R represents a hydrogen atom or a methyl group.

7. Process according to Claim 6, **characterized in that** the diene of formula (III) is chosen from 1-acetoxy-1,3-butadiene or 1-acetoxy-3-methyl-1,3-butadiene.

8. Process according to claim 1, **characterized in that** an acetylation reaction of the substituted anthraquinones thus obtained is carried out, followed if necessary by an oxidation reaction, in order to obtain the rheins of general formula (IV) 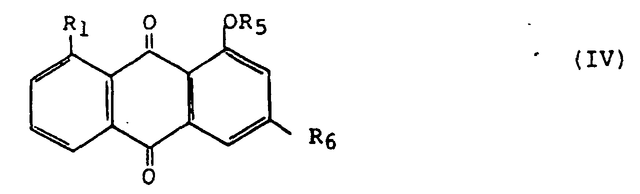 in which R₅ represents an acetyl group and R₆ represents a -CO₂R' group where R' is a hydrogen atom or a linear or branched alkyl group containing 1 to 5 carbon atoms.
